# EUROPEAN PATENT SPECIFICATION

(11) **EP 2 416 842 B1**
(45) Date of publication and mention of the grant of the patent: **05.07.2017**
(21) Application number: 10709672.9
(22) Date of filing: 17.03.2010
(51) Int. Cl.: A61N 1/05, A61B 17/34, A61M 25/01, A61M 25/00, A61M 25/10, A61N 1/36, A61B 17/00

(54) **ELECTRODE IMPLANTATION TOOL**
ELEKTRODENIMPLANTATIONSWERKZEUG
OUTIL DESTINÉ À L'IMPLANTATION D'ÉLECTRODES

(30) Priority: 17.03.2009 US 160763 P
(43) Date of publication of application: 15.02.2012
(73) Proprietor: AMS Research Corporation, Minnetonka, MN 55343 (US)
(72) Inventor: WATSCHKE, Brian, P., Minnetonka MN 55343 (US); BUYSMAN, John, J., Minnetonka MN 55343 (US); GINDELE, Paul, J., Minnetonka MN 55343 (US); MOOSAI, Shiva, P., Minnetonka MN 55343 (US)
(74) Representative: Vossius & Partner Patentanwälte Rechtsanwälte mbB
(86) International application number: PCT/US2010/027643
(87) International publication number: WO 2010/107900

(56) References cited:
- GB-A- 2 001 532
- GB-A- 2 001 532
- US-A- 5 902 331
- US-A- 5 902 331
- US-A1- 2004 039 371
- US-A1- 2004 039 371
- US-A1- 2005 055 063
- US-A1- 2005 055 063
- US-A1- 2008 009 914
- US-A1- 2008 009 914

## Description

### BACKGROUND

An estimated 51 million women (17 million of them in the U.S. alone) cope with urinary incontinence. Urinary incontinence is the medical term used to describe the condition of not being able to control the flow of urine from your body. Incontinence usually occurs because the urethra cannot close tightly enough to hold urine in the bladder.

Implantable electronic stimulator devices, such as neuromuscular stimulation devices, have been disclosed for use in the treatment of various pelvic conditions, such as urinary incontinence, fecal incontinence and sexual dysfunction. Such devices generally include one or more electrodes that are coupled to a control unit by electrode leads. Electrical signals are applied to the desired pelvic tissue of the patient through the electrode leads in order to treat the condition of the patient. The electrode leads are typically secured to the tissue using an anchor in the form of a helical coil. Exemplary implantable electronic stimulator devices and uses of the devices are disclosed in U.S. Patent Nos. 6,354,991, 6,652,449, 6,712,772 and 6,862,480. U.S. Patent No. 5,902,331 shows an electrode implantation tool including a guide means, such as a guide wire, a cardiac lead engaging means, and a cardiac lead having a lead body. The lead engaging means is configured to slide along the guide wire and push the distal end of the lead body of the lead along as the lead engaging means is pushed by lead pusher. Once the guide wire is in place, the physician uses the pusher to drive the lead engaging means and the lead body into the patient. The lead engaging means follows the guide wire thereby delivering the stimulating end of the lead body to the targeted site. When the lead pusher is retracted, it pulls the cardiac lead engaging means out of the patient while leaving the electrode and the lead body within the patient. The guide wire is also removed to complete the implantation of the cardiac lead.

One challenge with using a neuromuscular stimulation device to treat urinary incontinence is getting the electrode implanted in the urinary sphincter, which is only a few millimeters thick. One method of implanting the electrodes in the urinary sphincter involves delivering the electrodes into the urinary sphincter through a periurethral incision using an introducer. The physician generally positions the electrodes based on feel, but the physician may be aided by the use of imaging, such as X-ray, MRI, fluoroscopy, etc.

Even with such imaging, multiple implantation attempts by the physician may be required before the electrodes are positioned properly. Additionally, with each implantation attempt, there is risk of urethra and bladder perforation.

While the invention is defined in claim 1, embodiments described herein provide solutions to these and other problems, and offer other advantages over the prior art.

### SUMMARY

Embodiments of the following disclosure are directed to an electrode implantation tool that is designed to assist in the accurate implantation of one or more electrodes in tissue of a patient. One embodiment of the electrode implantation tool (130) comprises a catheter guide and introducer support (140), and catheter guide (142) and an introducer (144). The catheter guide and introducer support comprises a first alignment member (146), a second alignment member (148), and a body member (150) that is attached to the first and second alignment members. The body member fixes the relative orientations of the first and second alignment members. The catheter guide is supported by the first alignment member and comprises an elongate body (152) having a distal end (154) and a channel (156) that is aligned with a longitudinal axis (158). The introducer comprises a sheath (166) that is supported by the second alignment member. The sheath defines a longitudinal axis (168). The first and second alignment members orient the longitudinal axis of the sheath at a predetermined angle (184) to the longitudinal axis of the catheter guide.

In accordance with another embodiment, the electrode implantation tool comprises a catheter guide and introducer support (140), a catheter guide (142) and an introducer (144). The catheter guide and introducer support comprises a first alignment member (146), a second alignment member (148) and a body member (150) attached to the first and second alignment members. The body member fixes the relative orientations of the first and second alignment members. The catheter guide is supported by the first alignment member. The introducer comprises a sheath (166) that is supported by the second alignment member. The sheath comprises a distal end (172) having a curved section (174).

Another embodiment of the disclosure is directed to a method of implanting an electrode (112) in the urinary sphincter (314) of a patient. In the method, an electrode implantation tool (130) is provided (300). In one embodiment, the tool comprises a catheter guide and introducer support (140), a catheter guide (142) and an introducer (144). The catheter guide and introducer support comprises a first alignment member (146), a second alignment member (148) and a body member (150) attached to the first and second alignment members. The catheter guide is supported by the first alignment member. The introducer comprises a sheath (166) that is supported by the second alignment member. In one embodiment of the method, a distal end (154) of the catheter guide is inserted (302) into the urethra (314) of the patient. A distal end (172) of the sheath is advanced (312) into the urinary sphincter. Next, an electrode (112) is fed (318) through the sheath and into the urinary sphincter. The sheath and the catheter guide are then removed from the patient leaving the electrode implanted within the urinary sphincter.

Other features and benefits that characterize embodiments of the present disclosure will be apparent upon reading the following detailed description and review of the associated drawings.

### BRIEF DESCRIPTION OF THE DRAWINGS

FIG. 1 is a side plan view of an exemplary electronic stimulator device, in accordance with the embodiments of the invention.
FIGS. 2-4 are simplified cross-sectional views of an electrode implantation tool, which is formed in accordance with embodiments of the invention.
FIG. 5 is a simplified top view of a catheter guide and introducer support in accordance with embodiments of the invention.
FIGS. 6 and 7 are assembled and exploded isometric views, respectively, of an electrode implantation tool in accordance with embodiments of the invention.
FIG. 8 is an isometric view of the catheter guide and introducer support of FIGS. 6 and 7.
FIG. 9 is an assembled isometric view of the electrode implantation tool in accordance with embodiments of the invention.
FIG. 10 is a front plan view of the catheter guide and introducer support of FIG. 9 attached to a catheter guide.
FIG. 11 is an exploded isometric view of the catheter guide and introducer support of FIG. 9.
FIGS. 12 and 13 respectively are isometric and front plan views of a catheter guide and introducer support formed in accordance with embodiments of the invention.
FIGS. 14 and 15 respectively are isometric perspective assembled and exploded views of the electrode implantation tool in accordance with embodiments of the invention.
FIG. 16 is an isometric view of a stop member in accordance with embodiments of the invention.
FIG. 17 is a flowchart illustrating method of implanting electrodes in a urinary sphincter of a patient.
FIGS. 18-21 are simplified cross-sectional views of steps of a method of implanting electrodes in a urinary sphincter of a patient.
FIG. 22 is a simplified illustration of an exemplary location of the one or more electrodes implanted in the urinary sphincter.

### DETAILED DESCRIPTION OF ILLUSTRATIVE EMBODIMENTS

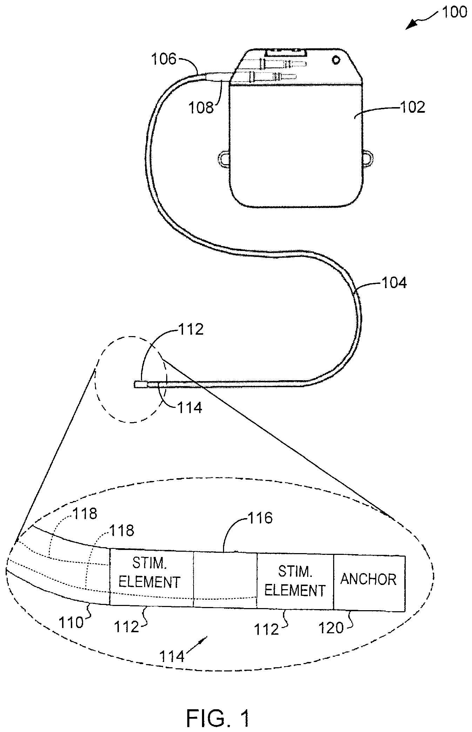

FIG. 1 is a side plan view of an exemplary electronic stimulator device 100, portions of which may be implanted in a patient using the devices and techniques of the present invention. The stimulator device 100 is configured for implantation into a pelvic region of a patient to provide muscle and/or nerve stimulation that is used to control and/or treat a pelvic condition of the patient, such as pelvic pain, urinary incontinence, fecal incontinence, erectile dysfunction or other pelvic condition that may be treated through electrical stimulation.

In one embodiment, the device 100 comprises a control unit 102 and one or more electrode leads 104, a proximal end 106 of which is coupled to the control unit 102 via a connector 108. Each electrode lead 104 comprises a lead body 110 and one or more stimulation elements or electrodes 112 attached to a distal end 114 of the lead body 110. In one embodiment, the electrodes 112 are separated from each other by an insulative portion or element 116. The lead body 110 insulates electrical wires 118 connecting the control unit 102 to the electrodes 112. The lead body 110 can be in the form of an insulating jacket typically comprising silicone, polyurethane or other flexible, biocompatible electrically insulating material. Additional electrode leads 104 or physiological sensors may be coupled to the control unit 102.

In one embodiment, the control unit 102 comprises circuitry for processing electrical signals received from the one or more electrodes 112 or physiological sensors (not shown). The control unit 102 is also configured to apply an electrical current or waveform to the tissue of the patient through the one or more electrodes 112 that are in contact with the tissue.

The distal end 114 of the electrode lead 104 can be anchored to pelvic tissue of the patient (e.g., urinary sphincter muscle) by means of a tissue anchor 120, such as a helical coil or other tissue anchor. The anchor 120 operates to secure the position of the electrodes 112 in the desired tissue of the patient.

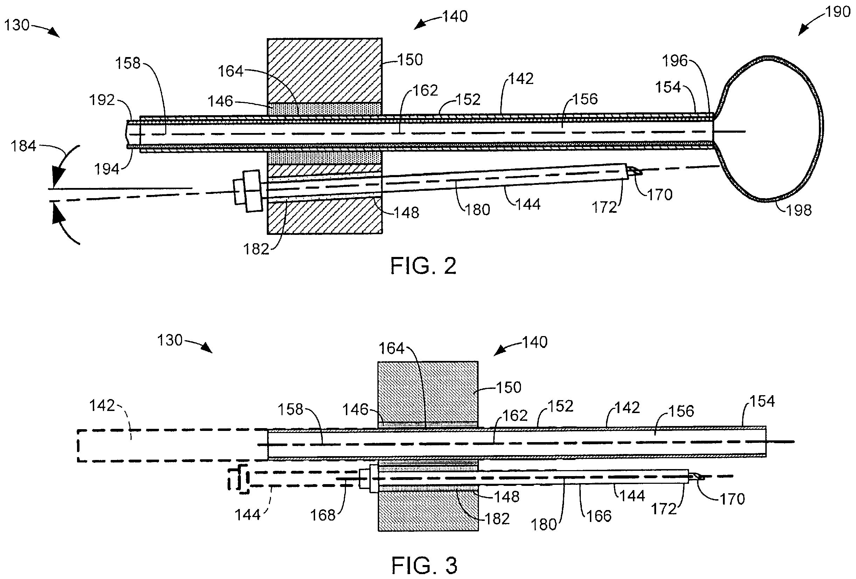

Embodiments of the invention are directed to an electrode implantation tool that is designed to assist in the accurate implantation of electrodes, such as electrodes 112, in tissue of the patient that is targeted for electrical stimulation. FIGS. 2-4 are simplified cross-sectional diagrams of an electrode implantation tool 130 formed in accordance with embodiments of the invention. Embodiments of the electrode implantation tool 130 generally include a catheter guide and introducer support 140, a catheter guide 142 and an introducer 144. Elements that are identified by the same or similar reference numbers in the figures generally correspond to the same or similar elements.

The support 140 generally comprises an alignment member 146, an alignment member 148 and a body member 150. The components are preferably formed of plastic or other suitable material.

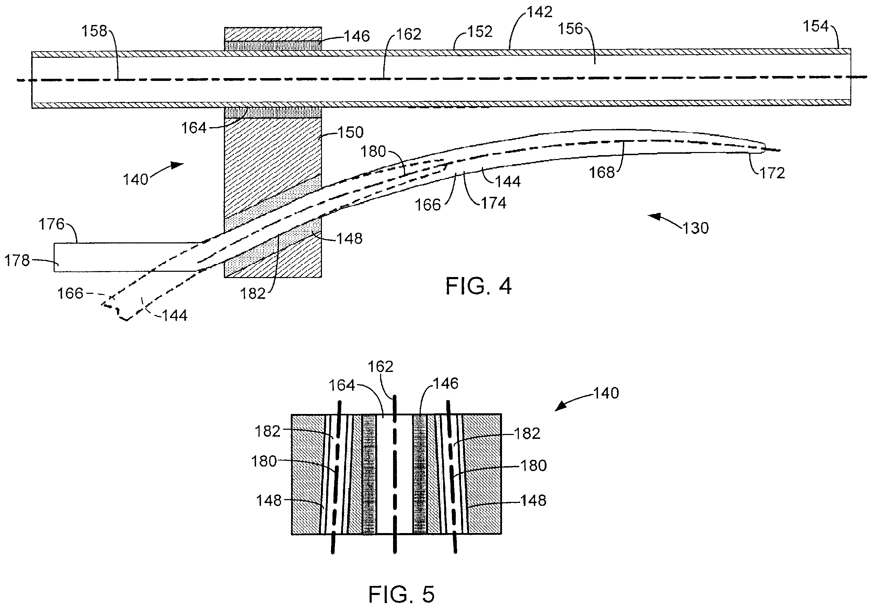

FIG. 5 is a schematic illustration of the body member 150 in accordance with embodiments of the invention. In one embodiment, the body member 150 is attached to the alignment members 146 and 148 and fixes the relative orientations of the alignment members 146 and 148. That is, movement of either of the alignment member 146 or the alignment member 148 produces a similar movement of the other alignment member 146 or 148 due to the connection between the alignment members 146 and 148 by the body member 150.

The catheter guide 142 is supported by the alignment member 146. The catheter guide 142 includes an elongate body 152 having a distal end 154 and a channel 156 that is generally aligned with a longitudinal axis 158. In one embodiment, the elongate body 152 comprises a rigid body that resists bending.

The alignment member 146, which may comprise several components, supports the catheter guide 142 such that the longitudinal axis 158 of the catheter guide 142 is substantially aligned with a central axis 162 (FIG. 5) of the alignment member 146. That is, the alignment member 146 engages the catheter guide 142 and directs the catheter guide 142 into a position that substantially aligns the longitudinal axis 158 of the catheter guide 142 with the central axis 162 of the first alignment member 146. In one embodiment, the alignment member 146 comprises a channel or bore 164 (hereinafter "channel") that defines the central axis 162, as illustrated in FIG. 5. The catheter guide 142 is supported in the channel 164, which directs the longitudinal axis 158 of the catheter guide 142 in substantial alignment with the central axis 162 through engagement with portions of the channel 164, as shown in FIGS. 2-4. The channel 164 may be formed in numerous ways while performing the aligning function.

In one embodiment, the location at which the alignment member 146 is attached to the catheter guide 142 is adjustable. In one embodiment, the catheter guide 142 can slide along the central axis 162 relative to the alignment member 146. Thus, the catheter guide 142 may be moved between a retracted position (shown in phantom) and an extended position while maintaining the longitudinal axis 158 of the catheter guide 142 in alignment with the central axis 162 of the alignment member 148, as shown in FIG. 3. The distal end 154 is positioned closer to the alignment member 146 when in the retracted position than when the catheter guide is in the extended position.

According to the invention, the introducer 144 includes a sheath 166 that generally defines a longitudinal axis 168 of the introducer 144. One embodiment of the introducer 144 also includes an introducer needle 170 (FIGS. 2 and 3) that is slidably received within the sheath 166 and assists in the feeding of a distal end 172 of the introducer 144 through tissue. The sheath 166 includes a lumen through which an electrode, such as electrode 112 of an electrode lead 104 (FIG. 1), may be passed through the distal end 172 for deployment into tissue of a patient. Alternatively, the needle 170 may include a lumen through which the electrode 112 may be deployed into tissue of a patient.

In one embodiment, the sheath 166 of the introducer 144 comprises a curved section 174 at the distal end 172, as illustrated in FIG. 4. The longitudinal axis 168 extending through the curved section 174 conforms to the curvature of the curved section 174. In one embodiment, the curved section 174 has a radius of approximately 2.5 inches. In one embodiment, the sheath 166 includes a straight section 176 at a proximal end 178.

The introducer 144 is supported by the alignment member 148. In one embodiment, the alignment member 148 supports the introducer 144 such that the longitudinal axis 168 is substantially aligned with a central axis 180 (FIG. 5) of the alignment member 148. That is, the alignment member 148, which may comprise several components, engages the introducer 144, such as the sheath 166, and directs the introducer 144 into a position that substantially aligns the longitudinal axis 168 of the introducer 144 with the central axis 180 of the alignment member 148. In one embodiment, when the sheath 166 of the introducer 144 includes the curved section 174, the central axis 180 of the alignment member 148 includes a curvature that matches that of the curved longitudinal axis 168, as shown in FIG. 4. In one embodiment, the alignment member 148 comprises a channel or bore 182 (hereinafter "channel") that defines the central axis 180, as illustrated in FIG. 5. The introducer 144 is supported in the channel 182, which directs the longitudinal axis 168 in substantial alignment with the central axis 180 through engagement with portions of the channel 182, as shown in FIGS. 2-4. The channel 182 may be formed in numerous ways while performing the aligning function.

In one embodiment, the sheath 166 of the introducer 144 is supported by the alignment member 148 such that the introducer 144 may slide along the central axis 180. This allows the introducer 144 to move between a retracted position (shown in phantom) and an extended position, as illustrated in FIGS. 3 and 4 while the longitudinal axis 168 of the portion of the sheath 166 within the alignment member 148 remains aligned with the central axis 180 of the alignment member 148.

In one embodiment, the alignment member 148 supports the introducer 144 such that the longitudinal axis 168 of the introducer 144 (and the central axis 180) is aligned at a predetermined angle 184 to the longitudinal axis 158 of the catheter guide 142 (and the central axis 162) supported in the alignment member 146, as illustrated in FIG. 2. In one embodiment, the predetermined angle 184 is in a range of 0-30 degrees. FIG. 3 illustrates the support of the introducer 144 by the alignment member 148 such that the longitudinal axis 168 is substantially parallel (predetermined angle 184 of 0 degrees) to the longitudinal axis 158. In accordance with one embodiment, the predetermined angle 184 is non-adjustable. In accordance with another embodiment, the first alignment member 146 and/or the second alignment member 148 are adjustably attached to the body member 150, which allows the predetermined angle 184 to be set as desired.

In one embodiment, the support 140 includes a pair of alignment members 148. In one embodiment, the alignment members 148 are positioned on opposing sides of the alignment member 146, as shown in FIG. 5. The pair of alignment members 148 allow the physician to select the side of the alignment member 146 to locate the introducer 144.

One embodiment of the electrode implantation tool 130 comprises a balloon catheter 190, which is shown in FIG. 2. The balloon catheter 190 includes a tube 192 having a proximal end 194 and a distal end 196. The balloon catheter 190 also includes a balloon 198 attached to the distal end 196 of the tube 192. The balloon 198 can be inflated (as shown) or deflated through the tube 192 in accordance with conventional techniques. In one embodiment, the balloon catheter 190 is a rigid balloon catheter. In one embodiment, the channel 156 of the catheter guide 142 is configured to receive the balloon catheter 190 and allow the balloon catheter 190 to be fed through the channel 156 and delivered to a desired target location.

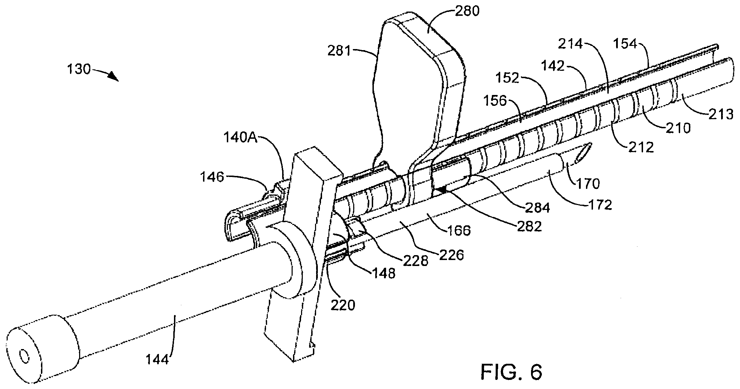

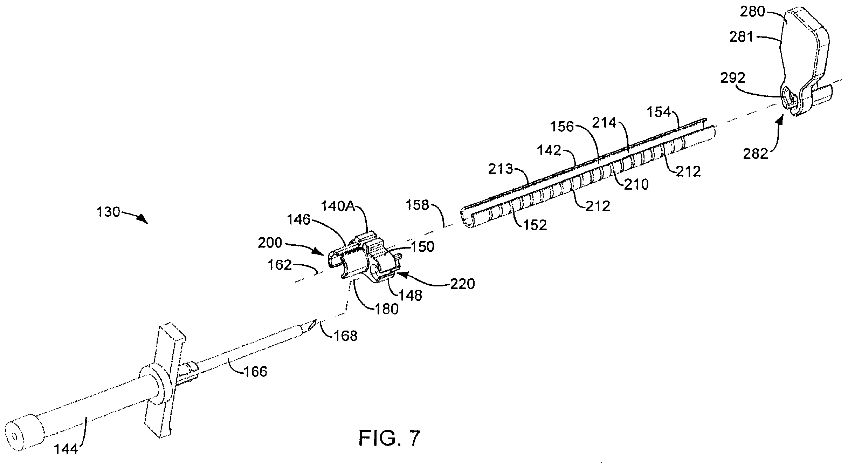

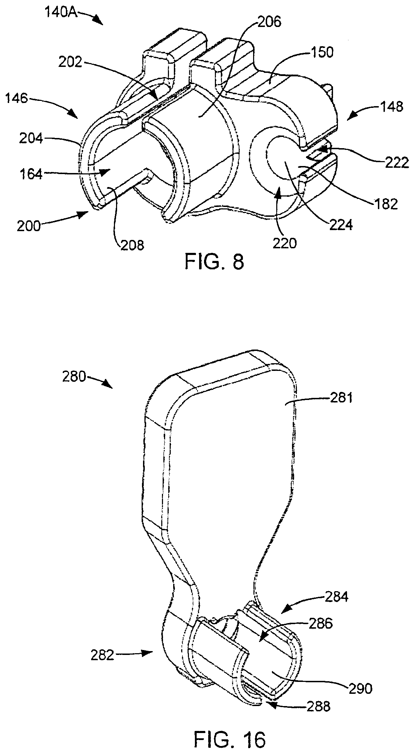

FIGS. 6 and 7 are assembled and exploded isometric views, respectively, of an electrode implantation tool 130 that includes a catheter guide and introducer support 140A in accordance with embodiments of the invention. FIG. 8 is an isometric view of the support 140A. As discussed above, the catheter guide 142 is supported by the first alignment member 146 and the introducer 144 is supported by the second alignment member 148. In one embodiment, alignment member 146 comprises a cylindrical portion 200 that generally defines the channel 164. In one embodiment, the cylindrical portion extends from the body member 150 in the direction of the central axis 162. In one embodiment, the cylindrical portion extends through the body member 150. In one embodiment, the cylindrical portion 200 includes a slot 202 separating the cylindrical portion 200 into first and second cylindrical sections 204 and 206. In one embodiment, the slot 202 extends in the direction of the central axis 162, as shown in FIG. 7.

A radius associated with an interior wall 208 (FIG. 8) of the cylindrical portion 200 measured from the central axis 162 approximately matches, or is slightly less than, the radius associated with the exterior surface 210 of the elongate body 152 of the catheter guide 142. The channel 164 allows the cylindrical sections 204 and 206 to flex in response to the reception of the catheter guide 142. This allows for a snug fit between the interior wall 208 and the exterior surface 210 and produces frictional resistance to sliding movement between catheter guide 142 and the alignment member 146.

In accordance with one embodiment, the exterior surface 210 of the catheter guide 142 includes a plurality of notches 212 that are spaced from each other along the longitudinal axis 158, as shown in FIGS. 6 and 7. In one embodiment, the alignment member 146 includes a protuberance that is configured to extend into one of the notches 212, when properly aligned, to resist relative movement between the catheter guide 142 and the body member 150 along the central axis 162. Thus, the notches 212 and the protuberance operate to provide the body member 150 a series of predefined position settings along the length of the catheter guide 142. In one embodiment, the notches 212 are equally spaced from each other. In one embodiment, the spacing between the notches 212 is 0.1-1.0 cm. In one embodiment, the elongate body 152 includes indicia indicating a distance from the tip of the distal end 154 of the catheter guide 142.

In one embodiment, the elongate body 152 of the catheter guide 142 comprises a cylindrical portion 213 that is coaxial to the longitudinal axis 162 and defines the channel 156. In one embodiment, the cylindrical portion 213 includes a slot 214 that is substantially parallel to the longitudinal axis 158, as shown in FIGS 6 and 7. One purpose of the slot 214 of the catheter guide 142 and the slot 202 of the first alignment member 146 is to allow for the insertion of the balloon catheter 190 into the channel 156 of the catheter guide after the tool 130 has been assembled.

As discussed above, the introducer 144 is supported by the alignment member 148. In one embodiment, the alignment member 148 comprises a cylindrical portion 220 that generally defines the channel 182 and supports the introducer 144, such as the sheath 166, in alignment with the central axis 180, as shown in FIGS. 6 and 7. In one embodiment, the cylindrical portion 220 includes a slot 222, as shown in FIG. 8. In one embodiment, a radius associated with an interior wall 224 of the cylindrical portion 220 measured from the central axis 180 approximately matches, or is slightly less than, the radius associated with the exterior surface 226 of the sheath 166 or other component of the introducer 144 that is connected to, or supports the sheath 166, such as component 228. This allows for a snug fit between the interior wall 224 and the exterior surface 226 and produces frictional resistance to sliding movement between the sheath 166 of the introducer 144 and the alignment member 148.

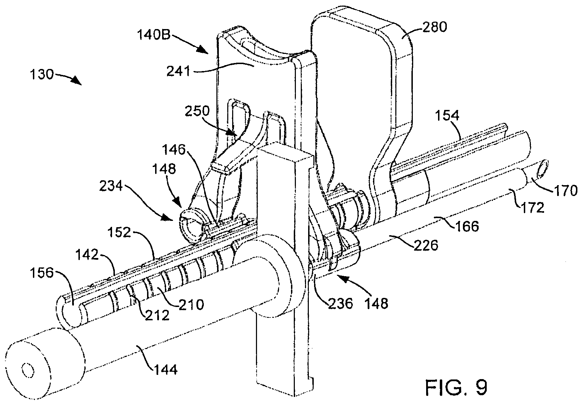

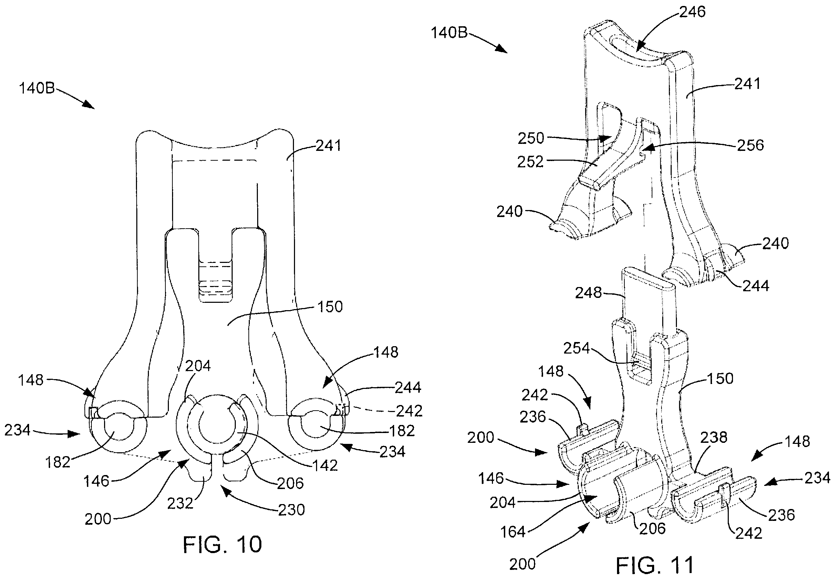

FIGS. 9-11 illustrate an electrode implantation tool 130 that includes a catheter guide and introducer support 140B in accordance with embodiments of the invention. FIG. 9 is an assembled isometric view of the electrode implantation tool 130, FIG. 10 is a front plan view of the support 140B attached to the catheter guide 142 and FIG. 11 is an exploded isometric view of the support 140B. The alignment member 146 of the support 140B is similar to the alignment member 146 of the support 140A, as shown in FIGS. 10 and 11. In one embodiment, the alignment member 146 includes a slot 230 extending substantially parallel to the axis 162 through a bottom portion 232 of the support 140B.

The alignment member 148 of the support 140B comprises a cylindrical portion 234 that generally defines the channel 182 that receives the sheath 166 of the introducer 144, or other component of the introducer 144, and supports the introducer 144 in alignment with the central axis 180, as discussed above. In one embodiment, the cylindrical portion 234 comprises a first portion 236 that is attached to the body member 150 through an arm 238 and a second portion 240, which is attached to a member 241. In one embodiment, the member 241 is removably attachable to the body member 150, as illustrated in FIG. 11. In one embodiment, the portions 236 and 240 include latching members 242 and 244, which interconnect to secure the portion 236 to the portion 240.

One embodiment of the member 241 includes a slot 246 that is configured to receive a tab 248 attached to the body member 150. In one embodiment, the support 140B includes a connector 250 comprising a component 252 attached to the member 241 and a component 254 attached to the body member 150 that engage each other to facilitate connecting the member 241 to the member 150, and the portion 236 to the portion 240. In one embodiment, the connector 250 is a latch and the connecting portion 252 is a flexible member comprising a groove 256 that receives the component 254 in the form of a protuberance to facilitate securing the member 241 to the body member 150, as shown in FIG. 11. The reception of the tab 248 within the slot 246 ensures proper alignment between the portions 236 and 240 of the cylindrical portion 234.

In accordance with one embodiment, the support 140B includes a pair of the alignment members 148, as shown in FIGS. 9-11. In one embodiment, the pair of alignment members 148 are positioned on opposing sides of the alignment member 146.

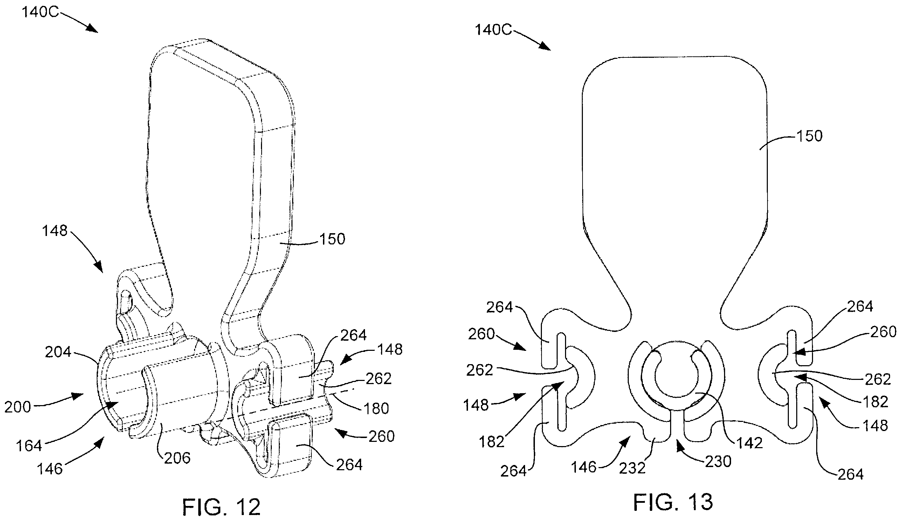

FIGS. 12 and 13 are isometric and front plan views of a catheter guide and introducer support 140C formed in accordance with embodiments of the invention. The plan view of FIG. 13 also includes the catheter guide 142. One embodiment of the support 140C comprises an alignment member 146 that is generally formed in accordance with the alignment member 146 of the support 140B.

In one embodiment, the alignment member 148 of the support 140C comprises a cylindrical portion 260 that generally defines the channel 182 that receives the sheath 166 of the introducer 144, or other component of the introducer 144, and supports the introducer 144 in alignment with the central axis 180 (FIG. 12), as discussed above. The cylindrical portion 260 includes an interior wall 262 that is generally parallel to the central axis 180 and directs the introducer 144 such that the longitudinal axis 168 is substantially aligned with the central axis 180. In one embodiment, the alignment member 148 includes one or more tabs 264 that flex and apply a spring force that presses the introducer 144 against the interior wall 262 and secures the introducer 144 to the alignment member 148 and generates frictional resistance to sliding movement between the sheath 166 and the alignment member 148.

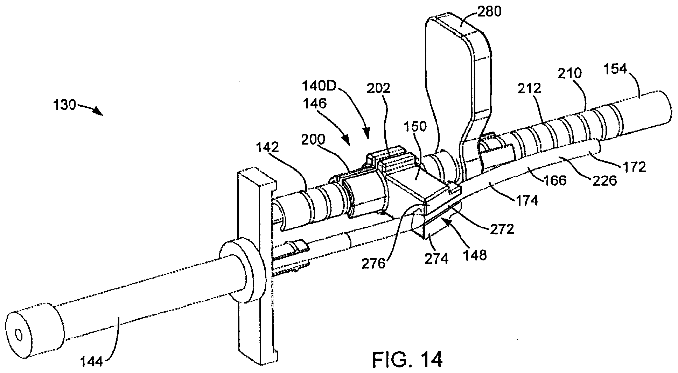

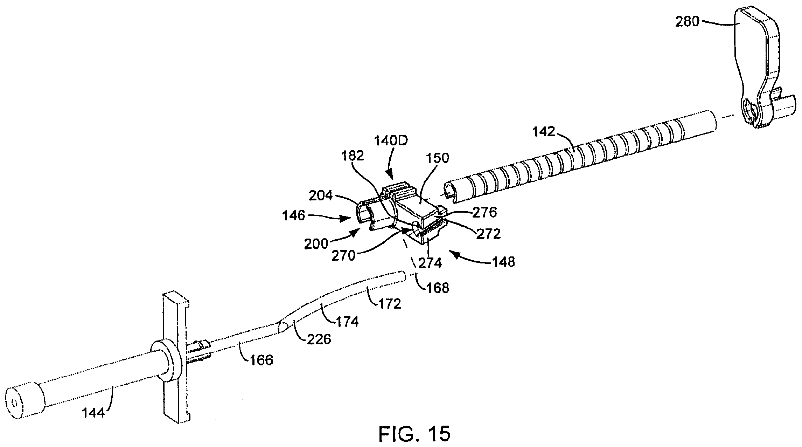

FIGS. 14 and 15 are isometric perspective assembled and exploded views of the electrode implantation tool 130 that includes a catheter guide and introducer support 140D in accordance with embodiments of the invention. One embodiment of the support 140D comprises an alignment member 146 that is similar to the alignment member 146 described above with regard to support 140A.

The alignment member 148 of the support 140D is configured to support the introducer 144 having the sheath 166 that includes the curved section 174 at the distal end 172, as discussed above with regard to FIG. 4. In one embodiment, the alignment member 148 comprises a curved cylindrical portion 270 that generally defines the channel 182 that receives the sheath 166 of the introducer 144, or other component of the introducer 144, and supports the introducer 144 in alignment with the central axis 180. In one embodiment, the diameter of the channel 182 is set to produce frictional resistance with the exterior surface 226 of the curved section 174 of the sheath 166. In one embodiment, the alignment member 148 includes a slot 272, which allows sections 274 and 276 of the cylindrical portion 270 to flex slightly to accommodate the sheath 166.

One embodiment of the electrode implantation tool 130 comprises a stop member 280 located on the distal end 154 side of the catheter guide 142, embodiments of which are illustrated in FIGS. 6, 7 and the isometric view of FIG. 16. The stop member 280 is generally used to limit the distance the distal end 154 of the catheter guide 142 is inserted into the patient. The stop member 280 comprises a body member 281 and an alignment member 282 that attaches to the exterior surface 210 of the catheter guide 142 and is formed similarly to the embodiments of the alignment member 146 described above. In one embodiment, the alignment member 282 comprises a cylindrical portion 284 that is configured to receive the catheter guide 142, as shown in FIG. 6. In one embodiment, the cylindrical portion 284 comprises a slot 286 on a top side. In one embodiment, the cylindrical portion 284 comprises a slot 288 located on a bottom side of the cylindrical portion 284. The cylindrical portion 284 comprises an interior wall 290 that is configured to engage the exterior surface 210 of the catheter guide 142 and provide frictional resistance there-between. In one embodiment, the slot 288 extends substantially parallel to the longitudinal axis 158 of the catheter guide 142 when the stop member 280 is attached to the catheter guide 142. As with the alignment member 146, one embodiment of the stop member comprises a protuberance 292, shown in FIG. 7, that can be received within one of the notches 212 of the catheter guide 142 to resist movement of the stop member 280 relative to the catheter guide 142 along the axis 158.

Another embodiment of the invention is directed to the use of embodiments of the electrode implantation tool 130 described above to implant one or more electrodes in the urinary sphincter of a patient. The electrodes, such as electrodes 112 of an electrode lead 104 (FIG. 1), can be coupled to a control unit 102 to facilitate electrical stimulation of the urinary sphincter in the treatment of incontinence or other condition of the patient.

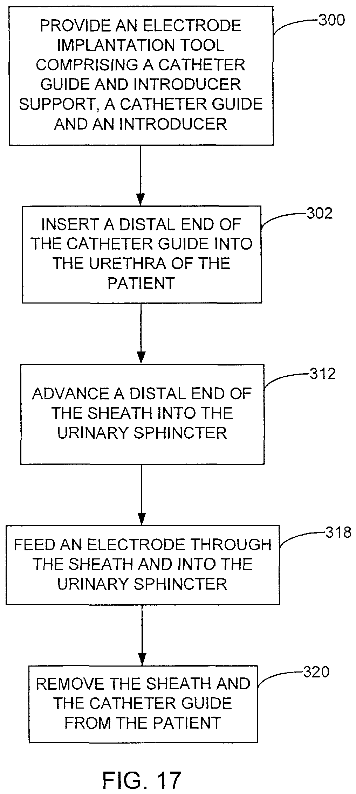

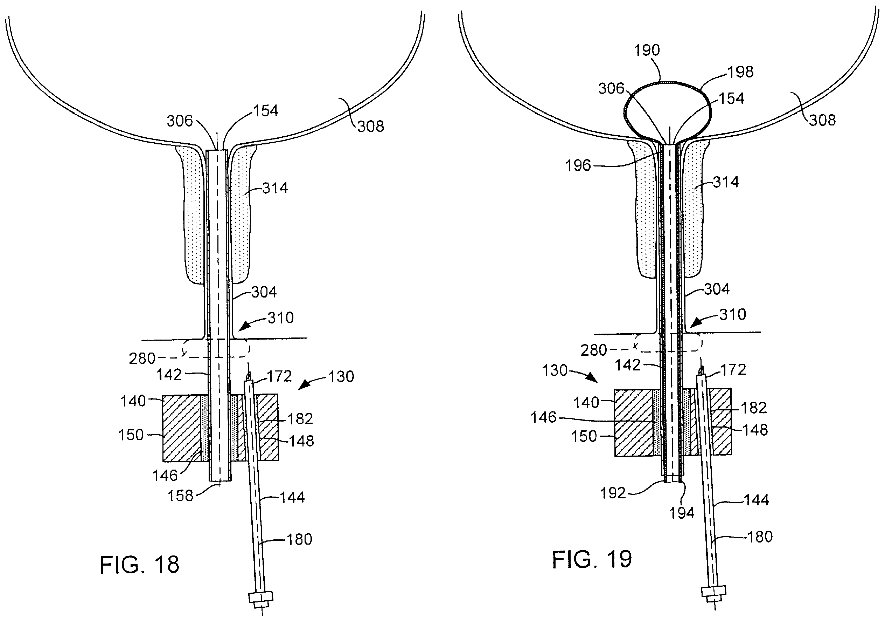

FIG. 17 is a flowchart illustrating a method of implanting electrodes in a urinary sphincter of a patient. At step 300, an electrode implantation tool 130 comprising a catheter guide and introducer support 140, a catheter guide 142 and an introducer 144 formed in accordance with one or more of the embodiments described above with reference to FIGS. 2-15 is provided. In one embodiment, the catheter guide and introducer support 140 comprises an alignment member 146, an alignment member 148 and a body member 150 that is attached to the alignment members 146 and 148. In one embodiment, the catheter guide 142 is supported by the alignment member 146. In one embodiment, the introducer 144 comprises a sheath 166 that is supported by the alignment member 148. Initially, the introducer 144 is supported by the alignment member 148 in a retracted position, as shown in FIG. 18. Alternatively, the introducer 144 may be inserted into the alignment member 146 when it is needed.

At 302 of the method, a distal end 154 of the catheter guide 142 is inserted into the urethra 304 of the patient, as illustrated in FIG. 18. In accordance with one embodiment, the distal end 154 of the catheter guide 142 is inserted into the urethra 304 until it is located proximate to the neck 306 of the bladder 308 of the patient. In accordance with one embodiment, the location of the bladder neck 306 is estimated based upon a statistical average of the distance from the urethral opening 310 to the bladder neck 306, which is approximately 4 cm. In one embodiment, a stop member 280 (shown in phantom) is attached to the catheter guide 142 at a predetermined location, such that the distal end 154 of the catheter guide 142 is positioned proximate the bladder neck 306 when the stop member 280 abuts the urethral opening 310. Thus, the stop member 280 limits the distance the distal end 154 of the catheter guide is inserted into the urethra 304.

The distal end 154 of the catheter guide 142 is positioned proximate to the bladder neck 306 of the patient is using a balloon catheter 190, which is shown in FIG. 19. The balloon catheter 190 comprises a tube 192 having a proximal end 194 and a distal end 196. A balloon 198 is attached to the distal end of the tube 192. In accordance with one embodiment of the method, the balloon catheter 190 is fed through the catheter guide 142, such as the channel 156, and into the bladder 308 of the patient while the balloon 198 is in a deflated state.

Alternatively, when the tube 192 is a rigid member (i.e., rigid balloon catheter), the channel 156 of the catheter guide 142 is sized to snap over the tube 192 such that an interference fit is created that prevents the tube 192 from sliding relative to the catheter guide 142 along the axis 158. The tube 192 is secured within the channel 156 with the deflated balloon 198 located just beyond the distal end 154. The distal end 154 of the catheter guide 142 can then be fed into the bladder 308 to position the balloon 198 within the bladder 308.

The balloon 198 is then inflated within the bladder 308 and the proximal end 194 of the tube 192, and/or the catheter guide 142, is pulled by the physician away from the distal end 196 to place the tube 192 in tension. This causes the balloon 198 to press upon the distal end 154 of the catheter guide 142 causing the catheter guide 142 to retract from the bladder 308 until the balloon 198 engages the bladder neck 306, as shown in FIG. 19. This process accurately positions the distal end 154 of the catheter guide 142 at the bladder neck 306.

In one embodiment, once the catheter guide 142 is in the desired location, the stop member 280 (shown in phantom) is slid along the catheter guide 142 toward the distal end 154 until it abuts the urethral opening 310, as shown in FIG. 19. The stop member 280 can operate like a clamp to secure the catheter guide 142 in the desired position within the urethra 134. The balloon catheter 190 can be removed along with the catheter guide 142 at the appropriate time.

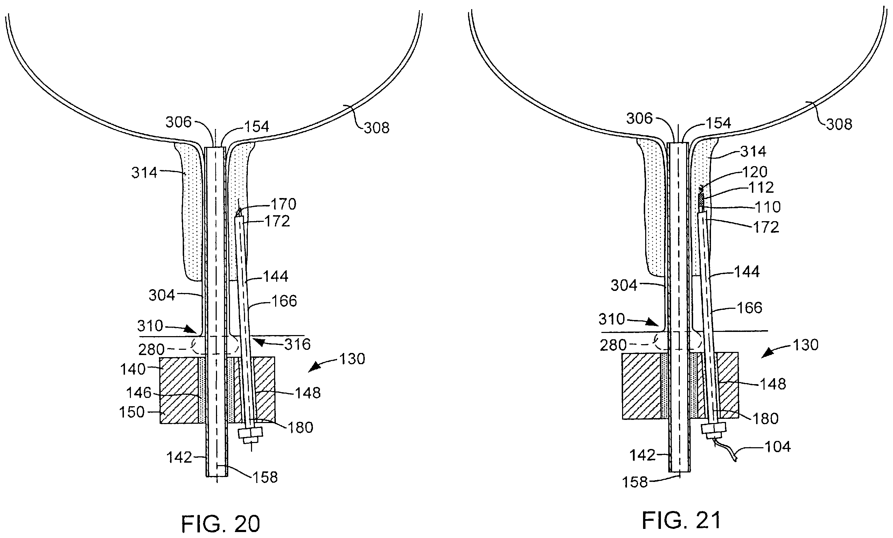

At 312, the distal end 172 of the introducer 144 is advanced into the urinary sphincter 314 of the patient, as shown in FIG. 20. In one embodiment, a periurethral incision 316 is first made, through which the distal end 172 is advanced toward the urinary sphincter 314. In accordance with one embodiment, the introducer 144 is moved relative to the alignment member 148 and the catheter guide 142 along the central axis 180 by sliding the introducer 144 through the channel 182 of the alignment member 148. The central axis 180 at which the alignment member 148 orients the sheath 166 of the introducer 144 and the distance the distal end 172 of the introducer is fed into the patient relative to the catheter guide 142 are selected to ensure that the distal end 172 does not pierce the urethra 304 or the bladder 308, but lands within the urinary sphincter 314. If necessary, the support 140 may be moved relative to the catheter guide 142 to position the introducer 144 in the position necessary to deliver the distal end 172 the desired distance to the target site within the urinary sphincter 314.

When the introducer 144 comprises the curved section 174 (FIGS. 4, 14 and 15), the distal end 172 is advanced from the retracted position into the urinary sphincter 314 along an arc corresponding to the curved longitudinal axis 168. In one embodiment, the central axis 180 and the curved section 174 are selected such that, as the distal end 172 travels along the arc path, the end 172 initially travels downward toward the catheter guide 142 until it reaches a minimum distance from the catheter guide 142, then the end 172 moves parallel to the catheter guide 142, and finally the end 172 travels slightly away from the catheter guide as it reaches the target site within the urinary sphincter 314.

At 318, at least one electrode 112, such as one attached to an electrode lead 104, is fed through the sheath 166, through the distal end 172, and into the urinary sphincter 314, as illustrated in FIG. 21. If an introducer needle 170 is used, it can be removed from the sheath 166 prior to the feeding step 318. In one embodiment, an anchor, such as anchor 120 (FIG. 1), anchors the electrode 112 to the urinary sphincter 314.

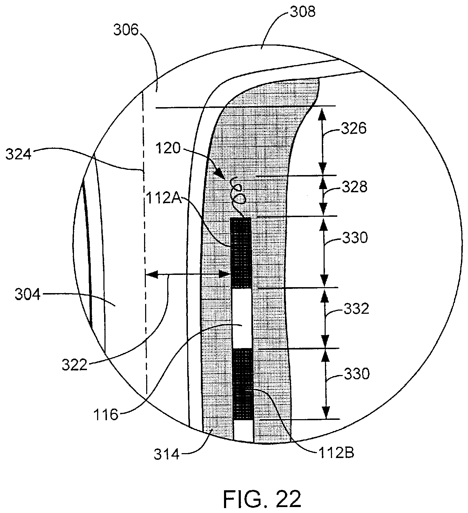

At step 320, the sheath 166 and the catheter guide 142 are removed from the patient leaving the at least one electrode 112 implanted within the urinary sphincter 314 of the patient. FIG. 22 is a simplified illustration of an exemplary location of one or more electrodes 112 implanted in the urinary sphincter 314 using one or more of the embodiments of the method described above. In general, it is desired that the distance 322 between a longitudinal axis 324 of the urethra 304 and the electrodes 112 be approximately 0.5 mm. It is also generally desired that the distance 326 separating the bladder neck 306 from the tissue anchor 120 be approximately 7.0 mm. The length 328 of a tissue anchor 120 in the form of a helical coil, is approximately 3.0 mm. In one embodiment, the distance separating the leading electrode 112A from the bladder neck 306 is approximately 1.0 cm (distance 326 + distance 328). The distances 330 corresponding to the approximate length of the leading electrode 112A and the trailing electrode 112B is approximately 4.0 mm. In one embodiment, the length 332 of the insulative component 116 is approximately 4.0 mm.

Although the present invention has been described with reference to preferred embodiments, workers skilled in the art will recognize that changes may be made in form and detail without departing from the scope of the invention. For example, although embodiments of the invention have been described as being used in to implant electrodes in a urinary sphincter of the patient, those skilled in the relevant art understand that the device may be designed to implant electrodes in other tissue, such as the anal sphincter, without departing from the scope of the invention.

## Claims

1. An electrode implantation tool (130) comprising:
a catheter guide and introducer support (140) comprising:
a first alignment member (146);
a second alignment member (148); and
a body member (150) attached to the first and second alignment members, wherein the body member fixes the relative orientations of the first and second alignment members;
a catheter guide (142) supported by the first alignment member, the catheter guide comprising an elongate body (152) having a distal end (154) and a channel (156) that is aligned with a longitudinal axis (158); and
an introducer (144) comprising a sheath (166) supported by the second alignment member, the sheath defining a longitudinal axis (168);
wherein the first and second alignment members orient the longitudinal axis of the sheath at a predetermined angle (184) to the longitudinal axis of the catheter guide
**characterised in that** the sheath has a lumen through which an electrode lead may be passed.

2. The tool of claim 1, wherein the predetermined angle is 0-30 degrees.

3. The tool of claim 1, wherein the first alignment member comprises a first channel (164) that defines a central axis (162), the first channel engages the catheter guide and directs the longitudinal axis of the catheter guide in substantial alignment with the central axis.

4. The tool of claim 3, wherein the first alignment member comprises a slot (202) extending substantially parallel to the central axis.

5. The tool of claim 3, wherein the second alignment member comprises a second channel (182) that defines a central axis (180) of the second alignment member, the second channel engages the introducer and directs the longitudinal axis of the introducer in substantial alignment with the central axis of the second alignment member.

6. The tool of claim 5, wherein the second alignment member comprises a slot (214) extending substantially parallel to the central axis of the second alignment member.

7. The tool of claim 5, wherein the second alignment member comprises a cylindrical portion (220) comprising a first portion (236) attached to the body member and a second portion (240) removably attached to the body member.

8. The tool of claim 1, wherein the elongate body comprises a cylindrical portion (213) that is coaxial to the longitudinal axis of the catheter guide and defines the channel, the cylindrical portion of the elongate body including a slot extending substantially parallel to the longitudinal axis of the catheter guide.

9. The tool of claim 1, further comprising a balloon catheter (190) comprising a tube (192) having proximal and distal ends (194, 196), and a balloon (198) attached to the distal end of the tube, wherein the tube is supported within the channel of the catheter guide.

10. The tool of claim 1, wherein the introducer comprises an introducer needle (170) within the lumen of the sheath.

## Patentansprüche

1. Elektrodenimplantationswerkzeug (130), das aufweist:
eine Katheterführungs- und ein Einführungshalterung (140), die aufweist:
ein erstes Ausrichtungselement (146);
ein zweites Ausrichtungselement (148); und
ein Körperelement (150), das an den ersten und zweiten Ausrichtungselementen befestigt ist, wobei das Körperelement die relativen Orientierungen der ersten und zweiten Ausrichtungselemente fixiert;
eine Katheterführung (142), die von dem ersten Ausrichtungselement gehalten wird, wobei die Katheterführung einen länglichen Körper (152) mit einem distalen Ende (154) und einem Kanal (156), der mit einer Längsachse (158) ausgerichtet ist, aufweist; und
einen Inserter (144), der eine Hülse (166) aufweist, die von dem zweiten Ausrichtungselement gehalten wird, wobei die Hülse eine Längsachse (168) definiert;
wobei die ersten und zweiten Ausrichtungselemente die Längsachse der Hülse in einem vorgegebenen Winkel (184) zu der Längsachse der Katheterführung orientieren,
**dadurch gekennzeichnet, dass** die Hülse ein Lumen hat, durch das ein Elektrodendraht geführt werden kann.

2. Werkzeug nach Anspruch 1, wobei der vorgegebene Winkel 0 - 30 Grad ist.

3. Werkzeug nach Anspruch 1, wobei das erste Ausrichtungselement einen ersten Kanal (164) aufweist, der eine Mittelachse (162) aufweist, wobei der erste Kanal in die Katheterführung eingreift und die Längsachse der Katheterführung im Wesentlichen in Ausrichtung mit der Mittelachse lenkt.

4. Werkzeug nach Anspruch 3, wobei das erste Ausrichtungselement einen Schlitz (202) aufweist, der sich im Wesentlichen parallel zu der Mittelachse erstreckt.

5. Werkzeug nach Anspruch 3, wobei das zweite Ausrichtungselement einen zweiten Kanal (182) aufweist, der eine Mittelachse (180) des zweiten Ausrichtungselements definiert, wobei der zweite Kanal in den Inserter eingreift und die Längsachse des Inserters im Wesentlichen in Ausrichtung mit der Mittelachse des zweiten Ausrichtungselements lenkt.

6. Werkzeug nach Anspruch 5, wobei das zweite Ausrichtungselement einen Schlitz (214) aufweist, der sich im Wesentlichen parallel zu der Mittelachse des zweiten Ausrichtungselements erstreckt.

7. Werkzeug nach Anspruch 5, wobei das zweite Ausrichtungselement einen zylindrischen Abschnitt (220) aufweist, der einen ersten Abschnitt (236), der an dem Körperelement befestigt ist, und einen zweiten Abschnitt (240) aufweist, der abnehmbar an dem Körperelement befestigt ist.

8. Werkzeug nach Anspruch 1, wobei der längliche Körper einen zylindrischen Abschnitt (213) aufweist, der koaxial mit der Längsachse der Katheterführung ist und den Kanal definiert, wobei der zylindrische Abschnitt des länglichen Körpers einen Schlitz umfasst, der sich im Wesentlichen parallel zu der Längsachse der Katheterführung erstreckt.

9. Werkzeug nach Anspruch 1, das ferner einen Ballonkatheter (190) aufweist, der ein Rohr (192) mit proximalen und distalen Enden (194, 196) und einen Ballon (198) aufweist, der an dem distalen Ende des Rohrs befestigt ist, wobei das Rohr in dem Kanal der Katheterführung gehalten wird.

10. Werkzeug nach Anspruch 1, wobei der Inserter eine Inserternadel (170) in dem Lumen der Hülse aufweist.

## Revendications

1. Outil d'implantation d'électrode (130) comprenant :
un support de dispositif d'introduction et de guide de cathéter (140) comprenant :
un premier élément d'alignement (146) ;
un second élément d'alignement (148) ; et
un élément de corps (150) fixé aux premier et second éléments d'alignement,
dans lequel l'élément de corps détermine les orientations relatives des premier et second éléments d'alignement ;
un guide de cathéter (142) supporté par le premier élément d'alignement, le guide de cathéter comprenant un corps allongé (152) ayant une extrémité distale (154) et un canal (156) qui est aligné avec un axe longitudinal (158) ; et
un dispositif d'introduction (144) comprenant une gaine (166) supportée par le second élément d'alignement, la gaine définissant un axe longitudinal (168) ;
dans lequel les premier et second éléments d'alignement orientent l'axe longitudinal de la gaine à un angle prédéterminé (184) vers l'axe longitudinal du guide de cathéter, **caractérisé en ce que** la gaine a une lumière à travers laquelle un fil d'électrode peut passer.

2. Outil selon la revendication 1, dans lequel l'angle prédéterminé est de 0-30 degrés.

3. Outil selon la revendication 1, dans lequel le premier élément d'alignement comprend un premier canal (164) qui définit un axe central (162), le premier canal met en prise le guide de cathéter et dirige l'axe longitudinal du guide de cathéter en alignement sensible avec l'axe central.

4. Outil selon la revendication 3, dans lequel le premier élément d'alignement comprend une fente (202) s'étendant sensiblement parallèlement à l'axe central.

5. Outil selon la revendication 3, dans lequel le second élément d'alignement comprend un second canal (182) qui définit un axe central (180) du second élément d'alignement, le second canal met en prise le dispositif d'introduction et dirige l'axe longitudinal du dispositif d'introduction en alignement sensible avec l'axe central du second élément d'alignement.

6. Outil selon la revendication 5, dans lequel le second élément d'alignement comprend une fente (214) s'étendant sensiblement parallèlement à l'axe central du second élément d'alignement.

7. Outil selon la revendication 5, dans lequel le second élément d'alignement comprend une partie cylindrique (220) comprenant une première partie (236) fixée à l'élément de corps et une seconde partie (240) fixée de manière amovible à l'élément de corps.

8. Outil selon la revendication 1, dans lequel le corps allongé comprend une partie cylindrique (213) qui est coaxiale par rapport à l'axe longitudinal du guide de cathéter et définit le canal, la partie cylindrique du corps allongé comprenant une fente s'étendant sensiblement parallèlement à l'axe longitudinal du guide de cathéter.

9. Outil selon la revendication 1, comprenant en outre un cathéter de ballonnet (190) comprenant un tube (192) ayant des extrémités proximale et distale (194, 196), et un ballonnet (198) fixé aux extrémités distales du tube, dans lequel le tube est supporté à l'intérieur du canal du guide de cathéter.

10. Outil selon la revendication 1, dans lequel le dispositif d'introduction comprend une aiguille d'introduction (170) à l'intérieur de la lumière de la gaine.
